(19) **Europäisches Patentamt / European Patent Office / Office européen des brevets**

(11) **EP 2 058 018 A1**

(12) # EUROPEAN PATENT APPLICATION
published in accordance with Art. 153(4) EPC

(43) Date of publication:
**13.05.2009 Bulletin 2009/20**

(21) Application number: **07806308.8**

(22) Date of filing: **30.08.2007**

(51) Int Cl.:
*A61M 1/36* (2006.01)      *B01J 20/26* (2006.01)
*D04H 1/42* (2006.01)

(86) International application number:
**PCT/JP2007/066831**

(87) International publication number:
**WO 2008/026667 (06.03.2008 Gazette 2008/10)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **31.08.2006 JP 2006235039**

(71) Applicant: **Toray Industries, Inc.**
**Tokyo, 103-8666 (JP)**

(72) Inventor: **SHIMAGAKI, Masaaki**
**Otsu-shi**
**Shiga 520-8558 (JP)**

(74) Representative: **Kador & Partner**
**Corneliusstrasse 15**
**80469 München (DE)**

(54) **ADSORPTION CARRIER CONTAINING COMPOSITE FIBER**

(57)   Disclosed is an adsorption carrier which can remove a cell occurring in the blood, preferably an activated leukocyte such as a granulocyte or a monocyte or a cancer cell, more preferably a cytokine present in an excessive amount, and which has a significantly increased cytokine-adsorbing ability compared to a conventional technique. The adsorption carrier comprises a fiber A having a diameter of 0.5 to 8 [mu]m inclusive and a fiber B having a diameter of 8 to 50 [mu]m inclusive, wherein the diameter of the fiber B is larger than that of the fiber A, and the fiber B comprises a sheath-core-type or island-sea-type composite fiber.

EP 2 058 018 A1

**Description**

Technical Field

The present invention relates to a novel adsorption carrier, specifically to an adsorption carrier suitable for blood-processing columns through which blood components are passed. The present invention also relates to a blood-processing column including the adsorption carrier of the present invention, the column being used as an adsorbent module suitable for adsorption and removal of cells and humoral factors contained in the blood.

Background Art

In recent years, various blood-processing columns have been studied. For example, columns for removal of leukocytes and granulocytes (Patent Documents 1 and 2), columns for adsorption of toxins and cytokines (Patent Documents 3 and 4), and columns for simultaneous adsorption of leukocytes and toxins (Patent Document 5) have been developed. These columns usually contain filters or adsorption carriers for removing and adsorbing target substances. Various materials and shapes of these filters or adsorption carriers are available, and each of them has merits and demerits. For example, in the leucocyte removal carrier (Patent Document 1) composed of a polyester nonwoven fabric, a leukocyte removal filter is realized by producing nonwoven fabric composed of fibers having a diameter of 3 $\mu$m or less. However, the carrier has a high bulk density, and tends to cause clogging of blood to be treated.

The adsorption carrier composed of cellulose acetate beads having a diameter of about 2 to 3 mm (Patent Document 2) less likely causes pressure drop, but the adsorption carrier is unsuitable for increasing the adsorptive surface area. Therefore, the adsorption carrier is not so efficient. However, it is difficult to decrease the particle diameter for increasing the adsorptive surface area because it causes increase of pressure drop of the blood to be treated.

The diameter of the fibers used in Patent Documents 3 and 4 is about 30 $\mu$m. In these documents, adsorption of toxins and cytokines are proposed, but function for cell adsorption is not imparted.

If the bulk density of the adsorption carrier is too high, the blood to be treated tends to cause clogging, and if the bulk density is too low, the adsorption carrier has poor form stability. Therefore, the bulk density should be from 0.05 to 0.15 g/cm$^3$, and is preferably from 0.10 to 0.15 g/cm$^3$, as disclosed in Patent Document 6. However, those having a bulk density of 0.05 to 0.10 g/cm$^3$ exhibits poor form stability, so that there has been developed no practical one having a bulk density of 0.10 to 0.15 g/cm$^3$. Also disclosed is a cell adsorbent composed of a sheath-core or islands-in-sea composite fibers having a diameter of 10 $\mu$m or less, and ordinary fibers having a diameter of 10 $\mu$m or more, wherein the composite fibers adsorb cells. However, the diameter of the composite fibers is so small that the adsorbed cells mask the adsorptive areas to deteriorate the adsorptivity within a short time.

Patent Document 1: Japanese Patent Application Laid-open No. 60-193468
Patent Document 2: Japanese Patent Application Laid-open No. 5-168706
Patent Document 3: Japanese Patent Application Laid-open No. 10-225515
Patent Document 4: Japanese Patent Application Laid-open No. 12-237585
Patent Document 5: Japanese Patent Application Laid-open No. 14-113097
Patent Document 6: Japanese Patent Application Laid-open No. 14-172163

Disclosure of the Invention

Object to Be Attained by the Invention

The present invention has been accomplished in view of the above-described problems, and an object of the present invention is to provide an adsorption carrier for removing cells, particularly activated leucocytes such as granulocytes and monocytes and cancer cells, from blood, the carrier having less pressure drop and good shape stability. Another object of the present invention is to provide an adsorption carrier having an improved capacity for removing excessive humoral factors such as cytokines and toxins, and an excellent adsorption and removal ability per unit volume.

Means for Attaining the Object

1. An adsorption carrier comprising fibers A having a diameter of 0.5 $\mu$m or more to 8 $\mu$m or less and fibers B having a diameter of 8 $\mu$m or more to 50 $\mu$m or less, the fibers B having a larger diameter than the fibers A, and the fibers B being sheath-core or islands-in-sea composite fibers.

2. The adsorption carrier according to item 1, wherein both of the fibers A and B are sheath-core or islands-in-sea composite fibers.

3. The adsorption carrier according to item 1 or 2, wherein the fibers A and/or B have amino groups at least on surfaces of the fibers.

4. The adsorption carrier according to item 3, wherein the amino groups are quaternary ammonium groups.

5. The adsorption carrier according to item 4, wherein the counter ions of the quaternary ammonium groups are substantially chlorine.

6. The adsorption carrier according to any one of items 1 to 5, wherein substances to be adsorbed are tissue-derived substances.

7. The adsorption carrier according to any one of items 1 to 6, which is used for flowing liquid and/or gas containing substances having a diameter of 1 $\mu$m or more as substances to be adsorbed.

8. The adsorption carrier according to any one of items 1 to 7, which is composed of at least two layers including a sheet material layer composed of the fibers A and B, and a net layer having a void of 10 mm$^2$ in any 100 mm$^2$ area.

9. The adsorption carrier according to any one of items 1 to 8, wherein the fibers A and/or B include a crosslinked structure at least on surfaces of the fibers.

10. The adsorption carrier according to any one of items 1 to 9, which has a bulk density of 0.02 to 0.5 g/cm$^3$.

11. The adsorption carrier according to any one of items 1 to 10, wherein the form of the sheet material is at least one selected from fabrics, knits, nonwoven fabrics, and porous materials

12. An adsorbent module packed with the adsorption carrier according to any one of items 1 to 11.

13. An adsorbent module comprising the adsorption carrier according to item 10 or 11 wound into a cylindrical shape, which is accommodated in a cylindrical container having a blood inlet and a blood outlet at the ends of the container.

Effects of the Invention

The adsorption carrier of the present invention has less pressure drop when the carrier passes blood components for use, and good shape stability. Therefore, the adsorption carrier is suitably used for various blood-processing columns. The adsorption carrier can dramatically increase introduction amount of adsorptive functional group on the surfaces of the fibers B which are sheath-core or islands-in-sea composite fibers having a diameter of 8 $\mu$m or more to 50 $\mu$m or less. In conventional techniques, when cells having a diameter of 0.5 $\mu$m or more to 8 $\mu$m or less and having adsorptive functional groups introduced on the fibers adsorb to the fibers A to which such cells easily adsorb, the adsorbed cells physically block the functional groups from humoral components, which results in insufficient adsorptivity for toxins and cytokines. Through separation of functions, the adsorption carrier of the present invention has higher ability in comparison with conventional techniques in simultaneous removal of excessive leukocytes and cancer cells, which are unnecessary for human body, and tissue-derived substances such as cytokines. Therefore, the adsorption carrier is useful for the blood-processing and treatment for autoimmune diseases, cancers, and allergies. In addition, the adsorption carrier allows miniaturization of adsorbers.

Best Modes for Carrying out the Invention

In the present invention, in order to achieve the above-described objects, dedicated research has been conducted to obtain an adsorption carrier which efficiently and selectively adsorbs and removes excessive cells contained in the blood such as leucocytes and cancer cells, and tissue-derived substances such as cytokines, and allows safe extracorporeal circulation. The present invention has been accomplished through improvement of the problems in the conventional adsorption carriers that clogging is easily occurred because the bulk density is too high and that unless the nonwoven fabric has form stability, clogging of blood or the like may occur eventually even if a nonwoven fabric having a low bulk density is obtained. In addition, the present invention allows introduction of a large number of specific functional groups capable of adsorbing tissue-derived substances such as cells and cytokines to the adsorption carrier without deforming the voids formed by fibers. More specifically, the adsorption carrier of the present invention has a lower bulk density than conventional techniques, and has form stability.

In addition to the cytokines described above, other examples of the tissue-derived substance include derived from living body, lipids, saccharides, and hormones such as chemotactic factors, antibodies, complements, and lymphokine. In particular, objects removed for structure analysis and pattern analysis, and target substances to be treated or the like become objects. In addition, bacteria, bacterial toxins, and viruses exerting adverse effects on the living body are also treated as tissue-derived substances. Examples of the cells include blood cells and tumor cells, and the objects are substances oozed into exudates such as blood, lymph, ascites, and pleural effusion. Cells, yeasts, and bacteria cultured in the study are also the object.

The adsorption carrier of the present invention includes at least two kinds of fibers: fibers A having a diameter of 0.5 $\mu$m or more to 8 $\mu$m or less, and fibers B having a diameter of 8 $\mu$m or more to 50 $\mu$m or less. The fibers A and B are preferably used by forming fibers into a sheet material. The fibers A having a diameter within the above-described range are effective for adsorption and removal of cells such as leukocytes and cancer cells. More specific diameter should be determined in consideration of the desired adsorption performance. For example, fibers used for the removal of granulocytes preferably have a diameter of 0.5 $\mu$m or more, more preferably from 1 $\mu$m to 8 $\mu$m. Fibers having a diameter of 0.5 $\mu$m to 4 $\mu$m are suitably used for the removal of lymphocytes. In order to selectively remove granulocytes in preference to lymphocytes, fibers having a diameter of 4 $\mu$m to 8 $\mu$m, more preferably from 4.5 to 8 $\mu$m are used. Combination of the fibers with other fibers having a diameter of less than 0.5 $\mu$m markedly improves the efficiency in removal of tissue-derived substances without significant change of the bulk density. Quantification and measurement of the hematocrit value of blood cells may employ, for example, XT-1800iV manufactured by Sysmex Corporation. The number of granulocytes is calculated in terms of the number of neutrophils.

However, when the sheet material made of the fibers A alone is formed into an adsorption carrier, form stability is hard to be maintained because of the small diameter of the fibers. The problem can be solved through the use of a sheet material composed of the fibers A and fibers B having a larger diameter than the fibers A. If form stability is insufficient even in a portion, clogging may occur during flow of blood or the like, so that it is preferable that the fibers A and B be thoroughly mixed and dispersed using, for example, a blender. The fiber diameter herein is measured as follows: ten small samples are randomly taken from the adsorption carrier, photographed with, for example, a scanning electron microscope at a magnification of 1000 to 3000, and 10 fibers from each sample, that is, 100 fibers in total are measured for their diameter, and the average is calculated; when the average is 10 $\mu$m or more, the first decimal place is rounded off, and when the average is less than 10 $\mu$m, the second decimal place is rounded off. When the diameters of the fibers A and B are little different and have large distributions, and the structures of the fibers are not different, it is difficult to distinguish the fibers A from B. However, the fibers A and B can be distinguished by measuring their diameters by the following procedure: when the distribution of the diameters of the fibers A and B forms two groups, the average diameter of the fibers of each distribution is determined, and each of the smaller one is defined as the diameter of the fibers A and B, respectively. When the fibers A and B are mixtures of fibers having different diameter distributions, the group having an average diameter of 0.5 to 8 $\mu$m is regarded as the fibers A, and that having an average diameter of 8 to 50 $\mu$m is regarded as the fibers B. When distributions of different fibers are partially overlapped, known peak dividing means is used.

In the present invention, the range of the diameter is applied not only to columnar fibers, but also to, for example, those having an oval, rectangular, or polygonal section. In the latter case, the area of the diagram formed by tracing the outermost layer is measured, and the diameter of a circle equivalent to the area is measured to determine the fiber diameter. When the pattern is, for example, a star having five projections, the area of the diagram linking the five projections is calculated, and the diameter of a circle equivalent to the area is defined as the diameter referred herein.

The fibers B are sheath-core or islands-in-sea composite fibers. In the islands-in-sea composite fibers, the islands may be sheath-core fibers. The composite fibers include three or more polymer compositions in the core, sheath, and sea thereof, and the characteristics of the polymers are effectively exhibited. In this case, specific functional groups to be introduced can be individually selected according to the combination of the polymers used, which allows introduction of two or more functional groups to respective polymers. For example, different functional groups can be introduced to the polymers used as the sheath and sea components, respectively.

In usual cases, functional groups can be easily introduced to brittle polymers such as polystyrene, but such polymers are hard to use because of the brittleness. However, these polymers can be formed into fibers in the form of sheath-core or islands-in-sea composite fibers, to which functional groups having a function of, for example, adsorbing and removing cytokines from the blood, are easily attached. Accordingly, when appropriate functional groups are attached to the fibers B which are sheath-core or islands-in-sea composite fibers having a diameter of 8 $\mu$m or more to 50 $\mu$m or less, the resultant adsorption carrier exhibits great effect in adsorption and removal of cytokines and the like. The diameter of the fibers B is more desirable to be from 12 $\mu$m or more to 50 $\mu$m or less to maintain the bulkiness of the adsorption carrier. If the fibers B have a diameter as large as 100 $\mu$m, they favorably keep a small bulk density and high form stability, but are poorly miscible with the fibers A because the diameter is too large. As a result of this, the fibers A are poorly dispersed, which leads to the impairment of the homogeneity of the carrier. In addition, the surface area of the carrier for adsorbing cytokines and the like cannot be efficiently increased. Therefore, the diameter of the fibers B is preferably 50 $\mu$m or less. On the other hand, the specific surface area of the carrier increases as the fiber diameter decreases. This is favorable for the resultant adsorption carrier, but the bulk density of the sheet material composed of the fibers A and B cannot be kept to be small. Therefore, the diameter of the fibers B is preferably 8 $\mu$m or more. The diameter of the fibers B is preferably from 15 $\mu$m or more to 40 $\mu$m or less from the above viewpoints, and even more preferably from 17 $\mu$m or more to 30 $\mu$m or less from the viewpoint of handle ability. The diameter of the fibers B is preferably larger than that of the fibers A. On the other hand, the fibers A are effective in adsorption and removal of cells such as leucocytes and cancer cells. More specifically, in the present invention, the adsorption carrier can be obtained in which the fibers A and B efficiently share the function of adsorbing and removing toxic components from the blood. If the fibers A alone are sheath-core or islands-in-sea composite fibers, the fibers A serve as the main portion for exhibiting effects of adsorbing and removing leucocytes or the like and cytokines, but the fiber diameter is so small that the adsorptive areas are easily masked mainly by the adsorbed cells, which results in the deterioration of the adsorptivity for cytokines within a short time.

It is thus more preferable that both of the fibers A and B be sheath-core or islands-in-sea fibers, because they have areas capable of providing functional groups, and thus adsorb more cytokines from the blood and others. In addition, adsorption of cells is expected to increase. The mixing percentage of the fibers A and B should be determined as follows. When the diameter of the fibers A is 5 $\mu$m or less, the mixing percentage of the fibers A is preferably 80 wt% or less, more preferably 70 wt% or less. This case particularly requires the bulkiness holding function of the fibers B. The percentage of the fibers A should be decreased when the diameter of the fibers B is 15 $\mu$m or less, and is preferably 60 wt% or less. The percentage of the fibers A is preferably 40 wt% or more, but clogging of the blood or the like may occur if the percentage is too high. When the diameter of the fibers A is more than 5 $\mu$m and the diameter of the fibers B is 15 $\mu$m or less, the percentage of the fibers A may be further decreased to about 20 wt%. When the diameter of the fibers B is more than 15 $\mu$m, the percentage of the fibers A may be varied in the range of 25 to 80 wt%. When the diameters of the fibers A and B are close to each other, the percentage of the fibers A may be from 1 to 99 wt% to achieve handle ability. The above-described range is not exclusive, and the optimum value may be determined in consideration of the intended performance according to the above-described procedure.

The adsorption carrier of the present invention is particularly preferably composed of multicore islands-in-sea composite fibers wherein the core is polypropylene (hereinafter referred to as PP), the sheath is polystyrene (hereinafter referred to as PS), and the sea is polyethylene terephthalate, or islands-in-sea composite fibers wherein the islands are PP and the sea is PS. The combination of the materials is arbitrary as long as the spinning property is good. The use of polystyrene as the sheath component is particularly preferable to facilitate the introduction of functional groups to the sheath structure. In this case, the amino group-containing functional groups can be readily introduced through amide methylation. In related art, cyclic peptides such as polymyxin B and polymyxin S, polyethyleneimine, and quaternary ammonium salts are introduced.

It is easy to introduce specific functional groups to aromatic polymers such as PS by utilizing the reactivity of aromatic rings. However, such polymers have a property which is difficult to be handled because of their problems such as brittleness, insufficient heat resistance in some case, and limitation on organic solvents for washing in production process. The problems of brittleness and heat resistance can be solved through the introduction of a crosslinked structure to the surface by formaldehyde or paraformaldehyde. The introduction of the crosslinked structure may be accomplished through the crosslinking of the adsorption carrier itself, or coating with other polymer or the like.

As described above, in the present invention, leucocytes and cancer cells can be removed mainly by the portion composed of the fibers A and B through adsorption or filtration. In addition to the leucocytes and cancer cells, tissue-derived substances such as cytokines can be adsorbed and removed by appropriately selecting the material and diameter of the fibers. In order to efficiently adsorb and remove tissue-derived substances such as cytokines in addition to leucocytes and cancer cells, it is preferable that specific functional groups be introduced and fixed to the adsorption carrier. Through appropriate selection of the material of the fibers composing the adsorption carrier, particularly its nonwoven fabric portion, the ability of adsorbing and removing tissue-derived substances such as cytokines can be imparted without introduction of specific functional groups. However, introduction of specific functional groups allows more efficient adsorption of tissue-derived substances.

The functional groups preferably have amino groups. Therefore, the fibers A and B preferably have amino groups at least on surfaces of the fibers. Fibers having amino groups fixed on their surfaces efficiently adsorb cytokines from the blood and others.

Specific examples of the amino groups include amino group-containing cyclic peptide residues, polyalkyleneimine residues, benzylamino groups, and primary, secondary, and tertiary alkylamino groups. Among them, preferable are amino group-containing cyclic peptide residues, polyalkyleneimine residues, and more preferable are amino group-containing cyclic peptide residues due to their high adsorptivity for tissue-derived substances.

More specifically, the amino group-containing cyclic peptide is not particularly limited as long as it is a cyclic peptide composed of two or more, more preferably four or more and 50 or less, more preferably 16 or less amino acids, and having one or more amino group at the side chain thereof. Specific examples thereof include, polymyxin B, polymyxin E, colistin, gramicidin S, or alkyl or acyl derivatives thereof.

The polyalkyleneimine residue referred herein is obtained by alkylation of a polyalkyleneimine, such as polyethyleneimine, polyhexamethyleneimine, or poly(ethyleneimine-decamethyleneimine) copolymer or some nitrogen atoms thereof with a single or mixed hydrocarbon halides such as n-hexyl bromide, n-decanyl bromide, or n-stearyl bromide, or acylation of the polyalkyleneimine with a fatty acid such as butyric acid, valeric acid, lauryl acid, myristic acid, linoleic acid, or stearyl acid.

The amino group to be introduced is preferably a quaternary ammonium group. The quaternary ammonium salt and/or linear amino group as the functional group to be fixed is preferably composed of ammonia or primary to tertiary amino group chemically bonded to a polymer. The primary to tertiary amino group preferably has 18 or less carbon atoms for one nitrogen atom when represented by the number of carbon atom to achieve high reactivity. Among primary to tertiary amino groups, those bonded to a quaternary ammonium group obtained from a tertiary amino group having 3 or more, preferably 4 or less carbon atoms for one nitrogen atom, and 18 or less, preferably 14 or less alkyl groups is favorable from the viewpoint of cytokine adsorptivity. Specific examples of the tertiary amino group include trimethylamine, triethylamine, N,N-dimethyl hexylamine, N,N-dimethyloctylamine, N,N-dimethyllaurylamine, and N-methyl-N-ethyl-hexylamine. In the present invention, the bonding density of the quaternary ammonium salts and linear amino groups varies according to the chemical structure and usage of the water-insoluble carrier. If the bonding density is too low, the carrier may fail to function, and if too high, the carrier after fixation has poor physical strength, and the function of the adsorbent tends to deteriorate. Therefore, the bonding density is preferably 0.01 mol or more, more preferably 0.1 mol or more, and preferably 2.0 mol or less, more preferably 1.0 mol or less for one repeating unit of the water-insoluble carrier.

Quaternarization of amino groups is achieved through introduction of amino groups under catalysis of an iodine-containing compound such as potassium iodide. Other conventional techniques may be suitably used. Although the mechanism is unknown, adsorptivity for cytokines may be suppressed when the concentration of residual iodine is high. Therefore, the counter ions of quaternary ammonium groups are preferably chlorine from the viewpoint of processability. As a more simple method for decreasing the concentration of residual iodine, it is preferable that iodine be replaced with chlorine through washing with a normal saline solution or a saline solution of any concentration. More specifically, in consideration of affinity with water, the most preferable method is treatment with a solution containing a chlorine compound (chloride).

As described above, the concentration of residual iodine is preferably low. When the amount of residual iodine in the adsorption carrier was 1.4 wt% or lower, adsorptivity for cytokines such as interleukin-6 (hereinafter referred to as IL-6) was successfully improved and stabilized. The form of residual iodine referred herein may be iodine or iodide ion, and examples thereof include iodine, iodide ion, and triiodide ion. When the adsorption carrier contains cations, the counter ions may be iodide ions or triiodide ions. When they are oxidized, iodine may deposit on the surface. Measurement of the amount of the residual iodine referred herein may employ any method, for example, elementary analysis, fluorescent X-ray analysis, or titration. However, when iodine remains not as ions but in the form of iodine molecules, and no drying step is conducted before the use of the adsorption carrier and vacuum drying is conducted only during preparation of the measurement samples, the iodine may sublimate, which hinders the accurate determination of the amount of residual iodine at the time of use of the adsorption carrier. Therefore, the adsorption carrier must not be dried during preparation of the sample for measuring the amount of residual iodine in the production process thereof. When the concentration of iodine ions as counter ions is particularly 1.4 wt% or less, or 98.6 wt% or more of the iodine ions are replaced with chlorine ions, or the concentration of other halide ions is 5 wt% or less, or 95 wt% or more of the halide ions is replaced with chlorine ions, the counter ions are substantially chlorine ions.

The form of the sheet material composed of the fibers A and B may include at least one selected from fabrics, knits, nonwoven fabrics, and porous materials. The controllable sizes of the voids between the fibers vary depending on the form. When the form is a nonwoven fabric, the voids between fibers can be controlled in a wide range. Therefore it is preferable for practical use.

In the present invention, the material of the fibers may be selected from known polymers such as polyamide, polyester, polyacrylonitrile, their derivatives, polyethylene, and PP. The material of the fibers A and B are as described above. When other fibers are included, they may be single fibers or sheath-core, islands-in-sea, or side-by-side composite fibers composed of these polymers. The sectional shape of the fibers may be circular or other shape. The adsorption carrier is usually produced by forming a sheet material in the above-described form, and introducing predetermined functional groups thereto. The sheet material may be produced by a known technique. Examples of the method for producing a nonwoven fabric include known methods for producing nonwoven fabrics such as wet process, carding process, air-laying process, spunbonding process, or melt blowing process.

When the sheet material is formed into, in particular, a nonwoven fabric, the structure preferably includes two or more layers including a net to improve form stability. The structure including two or more layers mainly refers to a laminated structure. The structure may be composed of two layers of a nonwoven fabric and a net, and is more preferably a sandwich structure composed of a net sandwiched between two layers of nonwoven fabric, that is, a sandwiched structure of nonwoven fabric-net-nonwoven fabric. In consideration of the below-described bulk density of the adsorption carrier, the structure may include more layers without affecting the pressure drop before and after the adsorption carrier during passage of a medium to be treated.

In the present invention, the material of the net may be selected from known polymers such as polyamide, polyester, polyacrylonitrile, their derivatives, polyethylene, and PP. As will be described later, when the net integrated with a nonwoven fabric is subjected to organic synthesis reaction for introducing functional groups, the material may be appropriately selected according to the type of the solvent and reaction temperature. In particular, from the viewpoints of biocompatibility and resistance to steam sterilization, the material is particularly preferably PP. When radiation sterilization is conducted, polyester or polyethylene is preferred.

When the net structure is composed of wound yarn or spun yarn made of a plurality of filaments, pressure drop may increase during passage of the medium to be treated such as blood through the wound yarns. Therefore, the net is preferably composed of monofilaments. In addition, it is easy to maintain mechanical strength of each monofilament.

The diameter of a monofilament is preferably from 50 $\mu$m or more to 1 mm or less, and the thickness of the net is preferably from 50 $\mu$m or more to 1.2 mm or less. The larger size may be possible, but is not preferable because the amount of adsorption carrier per unit volume decreases as the increase of the net size.

The net structure is not particularly limited, and may be, for example, a knot net, knotless net, or raschel net. The mesh shape is also not particularly limited, and may be, for example, rectangular, rhombus, or testudinal. For example, when the mesh shape of the net is square, positional relationship of the material of the net relative to sheet material is made so as to form an angle of 90° $\pm$ 10° relative to the major axis or short axis direction of the sheet material thereby improving the strength and handle ability of the stacked sheet materials.

Combination of a net with a nonwoven fabric achieves higher form stability. Therefore, the resultant adsorption carrier has a stable form even its bulk density is small. The net itself affects the pressure drop of the medium to be treated, so that the mesh of the net is desirably as large as possible. For the purpose, the net desirably has a void of 10 mm$^2$ or more in any 100 mm$^2$ area, and particularly preferably has about 3-mm mesh for good form stability and suitable use.

The thickness of a piece of the adsorption carrier is not particularly limited. When the adsorption carrier is a sheet material, the thickness is preferably from 0.1 mm or more to 10 cm or less from the viewpoint of handle ability. For example, when the adsorption carrier is integrated into a radial flow module such as TORAYMYXIN (registered trademark) manufactured by Toray Industries, Inc., the sheet-like adsorption carrier is wrapped around the center pipe, so that level difference tends to occur at the start and end points of wrapping. In this case, the thickness is preferably 1 cm or less. When the adsorption carrier is simply packed in a column in layers, the thickness of the adsorption carrier may be determined according to the column size. The total thickness of the adsorption carrier is preferably 2 mm or more. When the adsorption carrier is laminated, the variation in performance is easily reduced. In this case, the thickness of the adsorption carrier is preferably about 3 cm for easy handling, but is acceptable up to about 10 cm.

In the present invention, the bulk density of the adsorption carrier is preferably 0.02 g/cm$^3$ or more, more preferably 0.05 g/cm$^3$ or more, and preferably 0. 5 g/cm$^3$ or less, more preferably 0.15 g/cm$^3$ or less. The bulk density referred herein means the bulk density of a felted sheet material at the final stage after reaction by introduction of desired functional groups. As the increase of the bulk density, the ability of filtrating large substances such as leucocytes and cells improves. However, if the bulk density is too high, clogging tends to occur during blood circulation, so that the bulk density is preferably within the above-described range. A nonwoven fabric having a bulk density of more than 0.15 g/cm$^3$ can keep sufficient form stability without taking the form according to the present invention, or a laminated structure composed of a net and a nonwoven fabric. The bulk density is measured as follows. The adsorption carrier is cut into a small square sample (3 cm per side), and the thickness of the adsorption carrier is measured with a PP plate (5 cm per side and 1 mm thick) mounted thereon so as to be overlapped in the thickness direction. The plate is removed and mounted on the sample again, and the thickness of the adsorption carrier is measured. The operation is repeated five times, and the average thickness is calculated. The weight of the small sample is divided by the volume to calculate the bulk density. The calculation is conducted for five samples, and the average bulk density is calculated. When the sample has a net, after the completion of the above-described measurement, only the net is removed, and the net weight is subtracted from the weight of the sample of the sample to calculate the bulk density as described above.

The method for producing the adsorption carrier of the present invention in the form of a nonwoven fabric is described below. The fibers A and B are weighed to give an intended mixing percentage, and the mixture is passed through a carding machine, and thoroughly dispersed to make cotton like materials. The cotton like materials are weighed to give an intended basis weight, passed through a cross-lapper, and needle-punched to make a nonwoven fabric. The nonwoven fabric is stacked on a net, which has been separately produced, by a known web adhesion method such as thermal bonding, calendering, or needle-punching thereby making a laminated structure. In order to make a laminated structure, it is more preferable that a net be sandwiched prepunched cotton like materials, followed by punching to make an adsorption carrier having a layer structure of nonwoven fabric-net-nonwoven fabric. The method is so simple and suitable for continuous production. Alternatively, an adsorption carrier having a multilayer structure may be produced by stacking two-layer structures each composed of a net placed on one side of a prepunched cotton like materials.

The adsorbent module of the present invention may be produced by packing the adsorption carrier in a container, particularly preferably a cylindrical container.

The adsorbent module may be, for example, made by forming the adsorption carrier in sheet form, overlapping a plurality of layers of the adsorption carrier and packing the resultant in a column. In another example of column, an adsorption carrier is cylindrically wound around a core material or without a core material to make a cylindrical filter, and the filter is accommodated in a cylindrical container having a blood inlet and a blood outlet at the ends of the container. In another example of column, a hollow cylindrical filter composed of a cylindrically wound adsorption carrier with the both ends sealed is accommodated in a cylindrical container having an inlet and outlet for blood, the outlet for blood of the container is arranged at any one portion communicating with the periphery of the hollow cylindrical filter (the blood flows from the inside to the outside of the hollow cylindrical filter) or communicating with the inner circumference of the hollow cylindrical filter (the blood flows from the outside to the inside of the hollow cylindrical filter). In particular, in the column containing a hollow cylindrical filter when the outlet for blood of the container is arranged at a portion communicating with the inner circumference of the hollow cylindrical filter, most of inflammatory leucocytes in the blood is quickly and thoroughly removed by the large nonwoven fabric at the periphery of the cylindrical filter, and residual few inflammatory leucocytes are removed by the small nonwoven fabric at the inner circumference of the cylindrical filter. The method allows efficient removal of inflammatory leucocytes, and is thus most preferred.

The adsorption carrier of the present invention may be used for flowing liquid and/or gas containing substances having a diameter of 1 $\mu$m or more as substances to be adsorbed. Examples of the substances having a diameter of 1 $\mu$m or more include blood cells and plasma and the like. More specifically, the adsorption carrier of the present invention is suitable for medical use. Therefore, the adsorbent module according to the present invention is useful as an extracorporeal circulation column used for treatment or a perfusion column used for research.

EXAMPLES

[Measurement method]

(Fiber diameter)

Ten small samples were randomly taken from the adsorption carrier produced in each production example, photographed with, for example, a scanning electron microscope at a magnification of 1000 to 3000, and 10 fibers from each sample, that is, 100 fibers in total were measured for their diameter, and the average was calculated; when the average was 10 $\mu$m or more, the first decimal place was rounded off, and when the average was less than 10 $\mu$m, the second decimal place was rounded off.

When the cross section was oval, rectangular, or polygonal, the area of the diagram formed by tracing the outermost layer was measured, and the diameter of a circle equivalent to the area was measured to determine the fiber diameter. However, for example, when the cross section was star having five projections, the area of the diagram linking the five projections was calculated, and the diameter of a circle equivalent to the area was defined as the diameter referred herein.

(Bulk density)

The adsorption carrier produced in each production example was arbitrarily cut into small square samples (3 cm per side), and the thickness of the adsorption carrier was measured with a PP plate (5 cm per side and 1 mm thick) mounted thereon so as to be overlapped in the thickness direction. The plate was removed and mounted on the sample again, and the thickness of the adsorption carrier was measured. The operation was repeated five times, and the average thickness was calculated. The weight of the small sample was divided by the volume to calculate the bulk density. The calculation was conducted for five samples, and the average bulk density was calculated. When the sample had a net, after the completion of the above-described measurement, only the net was removed, and the net weight was subtracted from the weight of the sample to calculate the bulk density as described above.

(Measurement of blood cells)

Quantification and measurement of the hematocrit value of blood cells in the blood employed XT-1800iV manufactured by Sysmex Corporation. The number of granulocytes was calculated in terms of the number of neutrophils.

(Cytokine adsorption evaluation)

Cytokine adsorption was evaluated by the EIA method using a commercial kit IL-6 (manufactured by Kamakura Techno-Science Inc.).

$$\text{Cytokine adsorption rate (\%)} = [(\text{cytokine concentration in serum before shaking}) - (\text{cytokine concentration in serum after shaking})] / (\text{cytokine concentration in serum before shaking}) \times 100$$

[Production Example 1]

(Adsorption carrier 1)

Islands-in-sea composite fibers having 32 islands

(fibers A1) and another islands-in-sea composite fibers having 16 islands (fibers B1) were spun from the following components at a spinning speed of 800 m/minute and a draw ratio of 3.

(Fibers A1)

Island component: PP

Sea component: "copolymer polyester containing ethyleneterephthalate units as the main repeating units, and 3 wt% of sodium 5-sulfoisophthalate as the copolymerization component" (PETIFA)
Composite percentage (weight percentage):
island : sea = 80:20

(Fibers B1)

Island component: PP

Sea component: mixture of 90 wt% of PS and 10 wt% of PP
Composite percentage (weight percentage):
island : sea = 20:80
65 wt% of the fibers A1 and 35 wt% of the fibers B1 were thoroughly mixed and dispersed using TUFT BLENDER, and passed through a carding machine to produce a sheet material. The sheet material was passed through a cross-lapper, adjusted to a desired basis weight, and needle-punched to obtain an adsorption carrier in the form of a nonwoven fabric. Subsequently, the nonwoven fabric was treated with a sodium hydroxide aqueous solution (3 wt%) at 90°C to dissolve the sea components, whereby a nonwoven fabric was produced (adsorption carrier 1).

(Intermediate 1)

Thereafter, 3 g of paraformaldehyde was dissolved in a mixture of 600 mL of nitrobenzene and 390 mL of sulfuric acid at 20°C. The solution was cooled to 0°C, to which 75.9 g of N-methylol-$\alpha$-chloroacetamido was added, and dissolved therein at 5°C or lower. 5 g of the adsorption carrier 1 was immersed in the solution, and allowed to stand at room temperature for 2 hours. Thereafter, the fibers were taken out, and washed in an excess amount of chilled methanol. After thorough washing with the methanol, the fibers were washed with water, and dried to obtain 6.5 g of $\alpha$-chloroacetamidomethyl-modified PS fibers (intermediate 1).

(Adsorption carrier 1 having functional groups introduced)

50 g of N, N-dimethyloctylamine and 8 g of potassium iodide were dissolved in 400 ml of dimethylformamide (DMF), and 5 g of the intermediate 1 was immersed in the solution and heated for three hours in a bath at 85°C. After heating, the fibers were taken out and washed with methanol, and immersed in a 1 mol/L saline solution. The fibers after immersion were washed with water, and vacuum-dried to obtain 6.8 g of dimethyloctyl ammonium-modified fibers (adsorption carrier 1 having functional groups introduced: AC-1 (adsorption carrier-1)). The thickness of the carrier 1 was 1.8 mm.

[Production Example 2]

(Adsorption carrier 2)

Islands-in-sea composite fibers having 36 islands, the islands being sheath-core composite fibers (fibers A2) and fibers which are not islands-in-sea composite fibers or sheath-core composite fibers (fibers B2) were spun from the following components under the same spinning conditions as in Production Example 1.

(Fibers A2)

Island core component: PP

Island sheath component: mixture of 90 wt% of PS and 10 wt% of PP
Sea component: PETIFA
Composite percentage (weight percentage):
core : sheath : sea = 40:40:20

(Fibers B2)

Component: PP

Fiber diameter: 25 $\mu$m
65 wt% of the fibers A2 and 35 wt% of the fibers B2 were used to produce a nonwoven fabric under the same conditions as in Production Example 1 (adsorption carrier 2).

(Intermediate 2)

The adsorption carrier 2 was treated under the same conditions as in Production Example 1, whereby 6.6 g of $\alpha$-chloroacetamidomethyl-modified PS fibers (intermediate 2) were obtained.

(Adsorption carrier 2 having functional groups introduced)

6.9 g of adsorption carrier 2 (AC-2) having functional groups introduced was obtained from the intermediate 2 under the same conditions as in Production Example 1. The thickness of the adsorption carrier 2 having functional groups introduced was 1.9mm.

[Example 1]

50 ml of the blood (hematocrit value: 43%) was collected with heparin (heparin concentration: 10 U/ml) from a healthy volunteer, a natural human interleukin-6 manufactured by Kamakura Techno-Science Inc. was dissolved in the resultant to give a concentration of 500 pg/ml.
140 mg of AC-1 was packed in layers in an axial direction in a cylindrical column having an internal volume of 2 ml and a sectional diameter of 1 cm in the direction perpendicular to the axial direction. 25 ml of the blood was circulated for one hour at 37°C at a flow rate of 2.0 ml/min, and then the composition of the blood cells was analyzed by an automatic blood analyzer, and the amount of IL-6 was determined. The following quantification employed IL-6 quantification kit manufactured by Kamakura Techno-Science Inc. Table 1 lists the decrements (removal rates) of the lymphocytes, granulocytes, monocytes, and IL-6 in the blood after circulation in comparison with the blood before circulation. During the circulation, the increase of the blood pressure drop in the column (the drop of the blood pressure from the beginning to the end of passage through the column, the increase of 100 mmHg or more within one hour was unacceptable) did not become excessive. The maximum pressure drop in the column during circulation for one hour was 58 mmHg, which was measured at the end of the circulation.

[Comparative Example 1]

AC-2 made in Production Example 2 was packed in a column in the same manner as in Example 1 in the same amount, and remaining 25 ml of the blood collected in Example 1 was circulated through the column under the same conditions as in Example 1. Thereafter, the composition of the blood cells was analyzed with an automatic blood analyzer, and the amount of IL-6 was determined by the EIA method. Table 1 lists the removal rates for the respective substances, indicating that the decrement of IL-6 was only 43%. During the circulation, the pressure drop in the column did not become excessive. The maximum pressure drop in the column during circulation for one hour was 76 mmHg, which was measured at the end of the circulation. The configuration of the column was almost the same as that in Example 1, but the adsorptivity for cytokine was low, so that a smaller amount of cytokine was removed with the same amount of the adsorption carrier.

[Example 2]

190 mg of AC-1 and the same column as in Example 1 were used to make a column packed with a carrier in the same manner as in Example 1, and experimented under the same conditions as in Example 1. Table 1 lists the removal rates for the respective substances. The maximum pressure drop in the column during circulation for one hour was 68 mmHg at the end of the circulation.

[Comparative Example 2]

190 mg of AC-2 made in Production Example 2 was packed in a column in the same manner as in Example 2, and remaining 25 ml of the blood prepared in Example 2 was circulated through the column under the same conditions as in Example 2. Table 1 lists the removal rates for the respective substances. The maximum pressure drop in the column during circulation for one hour was 126 mmHg, which was measured at the end of the circulation. The pressure drop was higher than 100 mmHg as the criteria value, so that the column was unsuitable. The ability of removing leukocytes was almost the same as in Example 2, but the adsorptivity for cytokine was low, so that a smaller amount of cytokine was removed with the same amount of the adsorption carrier.

[Production Example 3]

(Adsorption carrier 3)

Islands-in-sea composite fibers having 32 islands, the islands being sheath-core composite fibers (fibers A3) and islands-in-sea composite fibers having 16 islands (fibers B3) were spun from the following components under the same spinning conditions as in Production Example 1.

(Fibers A3)

Island core component: PP

Island sheath component: mixture of 90 wt% of PS and 10 wt% of PP
Sea component: PETIFA
Composite percentage (weight percentage) : core : sheath : sea = 42:43:15

(Fibers B3)

Island component: PP

Sea component: mixture of 90 wt% of PS and 10 wt% of PP
Composite percentage (weight percentage): island : sea = 20:80
65 wt% of the fibers A3 and 35 wt% of the fibers B3 were thoroughly mixed and dispersed using TUFT BLENDER, and passed through a carding machine to produce a sheet material. Thereafter, a 2-mm mesh polyester net (thickness: 0.4 mm, monofilament diameter: 0.3 mm, basis weight: 75 g/m$^2$) was sandwiched between sheet materials in such a manner that the fiber direction of the net formed an angle of 5° to the axes of the sheet material, and then passed through a cross-lapper, adjusted to a desired basis weight, and needle-punched to obtain an adsorption carrier having a three-layer structure. Subsequently, the nonwoven fabric was treated with a sodium hydroxide aqueous solution (3 wt%) at 90°C to dissolve the sea components, whereby a nonwoven fabric was produced (adsorption carrier 3).

(Intermediate 3)

The adsorption carrier 3 was treated under the same conditions as in Production Example 1, whereby 6.8 g of α-chloroacetamidomethyl-modified PS fibers (intermediate 3) were obtained.

(Crosslinked fibers)

The adsorption carrier 3 was treated in the same manner as for the intermediate 3, except that N-methylol-$\alpha$-chloroacetamido was not added, and allowed to stand and react for 2 hours under room temperature in the same manner. Thereafter, the fibers were taken out, and washed in an excess amount of chilled methanol. After thorough washing with the methanol, the fibers were washed with water, and dried to obtain 5.5 g of PS crosslinked fibers (crosslinked fibers).
(Adsorption carrier 3 having functional groups introduced)
The intermediate 3 was treated under the same conditions as in Production Example 1, whereby 7.2 g of adsorption carrier 3 (AC-3) having functional groups introduced was obtained. The residual iodine was 0.9 wt% with reference to chlorine ions as determined by fluorescent X-ray analysis.
The obtained AC-3 included a net, so that it maintained a good shape with no deformation.

(Production Example 4)

(Adsorption carrier 4)

An adsorption carrier having a three-layer structure was obtained in the same manner as in Production Example 3, wherein the fibers A1 and PP fibers having a diameter of 19 $\mu$m (fibers B4) were used in place of the islands-in-sea composite fiber having 16 islands (fibers B1) used in Production Example 1. Subsequently, the nonwoven fabric was treated with a sodium hydroxide aqueous solution (3 wt%) at 90°C to dissolve the sea components, whereby a nonwoven fabric was produced (adsorption carrier 4).
The obtained adsorption carrier included a net, so that it maintained a good shape with no deformation.

[Example 3]

150 mg of AC-3 was packed in the same cylindrical column as that used in Example 1 in the same manner as in Example 1, and experimented under the same conditions as in Example 1. Table 1 lists the removal rates for the respective substances. The maximum pressure drop in the column during circulation for one hour was 52 mmHg, which was acceptable and measured at the end of the circulation.

[Comparative Example 3]

"Adsorption carrier 4" made in Production Example 4 was packed in the same cylindrical column as that used in Example 1 in the same manner as in Example 1, and tested in the same manner as Example 1 using remaining 25 ml of the blood prepared in Example 3.
Table 1 lists the removal rates for the respective substances. During the circulation, the pressure drop in the column did not become excessive. The ability of removing leukocytes was almost the same as in Example 2, but the adsorptivity for cytokine was low, so that a smaller amount of cytokine was removed with the same amount of the adsorption carrier. The maximum pressure drop in the column during circulation for one hour was 45 mmHg, which was measured at the end of the circulation.

[Example 4]

1 g of the "crosslinked fibers" was collected and immersed in a normal saline solution, and subjected to high-pressure steam sterilization at 121°C for 40 minutes. The surface of the adsorption carrier was composed of PS, but no melting or the like of the surface was found as observed with a scanning electron microscope, indicating that the adsorption carrier has high heat resistance. This is because the PS surface is crosslinked.

[Production Example 5]

(Adsorption carrier 5)

Islands-in-sea composite fibers having 32 islands, the islands being sheath-core composite fibers (fibers A5) and islands-in-sea composite fibers having 16 islands (fibers B5) were spun from the following components at a spinning rate of 800 m/minute and a draw ratio of 3.

(Fibers A5)

Island core component: PP

Island sheath component: mixture of 90 wt% of PS and 10 wt% of PP
Sea component: PETIFA
Composite percentage (weight percentage):
core : sheath : sea = 42:40:18

(Fibers B5)

Island component: PP

Sea component: mixture of 90 wt% of PS and 10 wt% of PP
Composite percentage (weight percentage):
island : sea = 20:80
A nonwoven fabric (adsorption carrier 5) composed of 62 wt% of the fibers A5 and 38 wt% of the fibers B5 was produced under the same conditions as in Production Example 3.

(Intermediate 5)

The adsorption carrier 3 was treated under the same conditions as in Production Example 1, whereby 6.8 g of α-chloroacetamidomethyl-modified PS fibers (intermediate 5) were obtained.

(Adsorption carrier 5 having functional groups introduced)

The intermediate 5 was treated under the same conditions as in Production Example 1, whereby 7.2 g of adsorption carrier 5 (AC-5) having functional groups introduced was obtained. The residual iodine was 0.8 wt% with reference to chlorine ions as determined by fluorescent X-ray analysis. [0048]
The obtained AC-5 included a net, so that it maintained a good shape with no deformation.

[Example 5]

50 ml of the blood of a healthy volunteer (hematocrit value: 41%) was heparin-collected (heparin concentration: 10 U/ml), a natural human interleukin-6 was dissolved in the resultant to give a concentration of 500 pg/ml.
160 mg of the "adsorption carrier 5 having functional groups introduced" was packed in the same cylindrical column as used in Example 1 in the same manner as in Example 1, and experimented under the same conditions as in Example 1. Table 1 lists the removal rates for the respective substances. The maximum pressure drop in the column during circulation for one hour was 81 mmHg, which was acceptable and measured at the end of the circulation.

[Production Example 6]

(Adsorption carrier 6)

Islands-in-sea composite fibers having 32 islands, the islands being sheath-core composite fibers (fibers A6) and islands-in-sea composite fibers having 16 islands (fibers B6) were spun from the following components under the same spinning conditions as in Production Example 1.

(Fibers A6)

Island core component: PP

Island sheath component: mixture of 90 wt% of PS and 10 wt% of PP
Sea component: PETIFA
Composite percentage (weight percentage) : core : sheath :
sea = 42:40:18
Sheath-core fiber diameter: 7.8 μm

(Fibers B6)

Island component: PP

Sea component: mixture of 90 wt% of PS and 10 wt% of PP
Composite percentage (weight percentage): island : sea = 20:80
A nonwoven fabric (adsorption carrier 6) composed of 30 wt% of the fibers A6 and 70 wt% of the fibers B6 was produced under the same conditions as in Production Example 3, the diameter of the sheath-core fibers being 7.9 $\mu$m.

(Intermediate 6)

Then, the adsorption carrier 3 was treated under the same conditions as in Production Example 1, whereby 6.8 g of $\alpha$-chloroacetamidomethyl-modified PS fibers (intermediate 6) were obtained.

(Adsorption carrier 6 having functional groups introduced)

The intermediate 5 was treated under the same conditions as in Production Example 1, whereby 7.2 g of adsorption carrier 6 (AC-6) having functional groups introduced was obtained. The residual iodine was 0.8 wt% with reference to chlorine ions as determined by fluorescent X-ray analysis.
The obtained adsorption carrier 6 having functional groups introduced included a net, so that it maintained a good shape with no deformation.

[Example 6]

The following test was conducted using remaining 25 ml of the blood collected in Example 5.
160 mg of the AC-6 was packed in the same cylindrical column as used in Example 1 in the same manner as in Example 1, and tested under the same conditions as in Example 1. Table 1 lists the removal rates for the respective substances. The maximum pressure drop in the column during circulation for one hour was 48 mmHg, which was acceptable and measured at the end of the circulation.

**Claims**

1. An adsorption carrier comprising fibers A having a diameter of 0.5 $\mu$m or more to 8 $\mu$m or less and fibers B having a diameter of 8 $\mu$m or more to 50 $\mu$m or less, the fibers B having a larger diameter than the fibers A, and the fibers B being sheath-core or islands-in-sea composite fibers.

2. The adsorption carrier according to claim 1, wherein both of the fibers A and B are sheath-core or islands-in-sea composite fibers.

3. The adsorption carrier according to claim 1 or 2, wherein the fibers A and/or B have amino groups at least on surfaces of the fibers.

4. The adsorption carrier according to claim 3, wherein the amino groups are quaternary ammonium groups.

5. The adsorption carrier according to claim 4, wherein the counter ions of the quaternary ammonium groups are substantially chlorine.

6. The adsorption carrier according to any one of claims 1 to 5, wherein substances to be adsorbed are tissue-derived substances.

7. The adsorption carrier according to any one of claims 1 to 6, which is used for flowing liquid and/or gas containing substances having a diameter of 1 $\mu$m or more as substances to be adsorbed.

8. The adsorption carrier according to any one of claims 1 to 7, which is composed of at least two layers including a sheet material layer composed of the fibers A and B, and a net layer having a void of 10 mm$^2$ in any 100 mm$^2$ area.

9. The adsorption carrier according to any one of claims 1 to 8, wherein the fibers A and/or B comprise a crosslinked structure at least on surfaces of the fibers.

10. The adsorption carrier according to any one of claims 1 to 9, which has a bulk density of 0.02 to 0.5 g/cm$^3$.

11. The adsorption carrier according to any one of claims 1 to 10, wherein the form of the sheet material is at least one selected from fabrics, knits, nonwoven fabrics, and porous materials.

12. An adsorbent module packed with the adsorption carrier according to any one of claims 1 to 11.

13. An adsorbent module comprising the adsorption carrier according to claim 10 or 11 wound into a cylindrical shape, which is accommodated in a cylindrical container having a blood inlet and a blood outlet at the ends of the container.

**INTERNATIONAL SEARCH REPORT**

| International application No. |
|---|
| PCT/JP2007/066831 |

A. CLASSIFICATION OF SUBJECT MATTER
*A61M1/36(2006.01)i, B01J20/26(2006.01)i, D04H1/42(2006.01)i*

According to International Patent Classification (IPC) or to both national classification and IPC

B. FIELDS SEARCHED

Minimum documentation searched (classification system followed by classification symbols)
A61M1/36, B01J20/26, D04H1/42

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2003-265606 A  (Toray Industries, Inc.), 24 September, 2003 (24.09.03), Full text (Family: none) | 1-13 |

☐ Further documents are listed in the continuation of Box C.    ☐ See patent family annex.

| | |
|---|---|
| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 03 October, 2007 (03.10.07) | 16 October, 2007 (16.10.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**EP 2 058 018 A1**

**REFERENCES CITED IN THE DESCRIPTION**

**Patent documents cited in the description**

- JP 60193468 A
- JP 5168706 A
- JP 10225515 A
- JP 12237585 B
- JP 14113097 B
- JP 14172163 B